# EUROPÄISCHE PATENTANMELDUNG

(11) **EP 1 120 390 A1**
(43) Veröffentlichungstag der Anmeldung: **01.08.2001**
(21) Anmeldenummer: 01100091.6
(22) Anmeldetag: 11.01.2001
(51) Int. Cl.: C07C 17/156, C07C 19/045, B01J 35/02, B01J 37/00

(54) **Verfahren zur Herstellung von 1,2-Dichlorethan**

(30) Priorität: 27.01.2000 DE 10003510
(71) Anmelder: BASF AKTIENGESELLSCHAFT, 67056 Ludwigshafen (DE)
(72) Erfinder: Meissner, Ruprecht, 67273 Weisenheim (DE); Hesse, Michael, Dr., 67549 Worms (DE); Walsdorff, Christian, Dr., 67061 Ludwigshafen (DE)
(74) Vertreter: Isenbruck, Günter, Dr.

(57) **Zusammenfassung**

Verfahren zur Herstellung von 1,2-Dichlorethan durch Oxychlorierung von Ethen in Gegenwart eines kupferhaltigen Festbett-Kontaktes aus einer Schüttung, im wesentlichen bestehend aus Katalysatorpartikeln, welche zumindest teilweise mit einer Aktivkomponente und gegebenenfalls einem Promotor imprägniertes Trägermaterial enthalten, wobei die Katalysatorschüttung im wesentlichen kein separates Inertmaterial zur Verdünnung enthält.

## Beschreibung

Die Erfindung betrifft ein Verfahren zur Herstellung von 1,2-Dichlorethan durch Oxychlorierung von Ethen, einen Katalysator, der in diesem Verfahren eingesetzt wird, sowie ein Verfahren zur Herstellung des Katalysators.

1,2-Dichlorethan wird heute in großtechnischem Maßstab durch Oxychlorierung von Ethen hergestellt. Dabei wird Ethen mit Chlorwasserstoff und Sauerstoff in Gegenwart eines Kupferkatalysators umgesetzt. Für die Umsetzung stehen Verfahren sowohl in der Gasphase an festen oder fluidisierten Kontakten als auch in flüssiger Phase mit gelösten Katalysatoren zur Verfügung, wobei großtechnische Anwendung nur Verfahren in der Gasphase gefunden haben, siehe Ullmanns Enzyklopädie der technischen Chemie, 4. Auflage 1975, Band 9, S. 428.

Die vorliegende Erfindung betrifft eine Verbesserung des Festbett-Verfahrens. Bei den Festbett-Verfahren bereitet die hohe Exothermie der Umsetzung Probleme, die bei höheren Belastungen, wie sie bei wirtschaftlicher Betriebsweise auftreten, zur Bildung sogenannter "hot spots" im Katalysatorbett führen kann. Die Bildung solcher "hot spots" muß unbedingt vermieden werden, da sie mit einer Desaktivierung des Katalysators durch Verdampfen des Kupfersalzes und einer Schädigung der Katalysatorformkörper einhergeht. Daher wird in den Festbettverfahren nach dem Stand der Technik eine Katalysatorschüttung mit abgestuftem Aktivitätsprofil verwendet, wobei die Aktivität der Katalysatorschüttung in Strömungsrichtung zunimmt. Dies wird üblicherweise dadurch erreicht, daß die Katalysatoren mit einem abgestuften Gehalt an Aktivkomponenten und gegebenenfalls Selektivpromotoren imprägniert werden. Zusätzlich hierzu wird nach dem Stand der Technik die Katalysatorschüttung zumindest in den Reaktorabschnitten, in denen die Triebkraft der Reaktion noch sehr hoch ist, also üblicherweise im vorderen Bereich der Katalysatorschüttung mit Partikeln aus einem inerten Material, beispielsweise aus gebrochenem Graphit, tablettiertem oder extrudiertem Graphitpulver oder nicht imprägniertem Katalysatorträger, verdünnt. Derartige Verfahren sind in den US 3,184,515 und US 2,866,830 beschrieben.

Der Einsatz inerter Verdünnungsmaterialien wie Graphit bringt jedoch als wesentlichen Nachteil einen erhöhten Druckverlust mit sich, der sich insbesondere bei Verwendung druckverlustarmer Katalysatorformkörper auswirkt und den Stoff- und Wärmetransport in, axialer Richtung behindert. Die Verarbeitung von reinem Graphit zu druckverlustarmen Formkörpern ist schwierig und relativ teuer. Der Einsatz von nicht imprägniertem Träger als Verdünnungsmaterial empfiehlt sich wegen der schlechteren Wärmeleitfähigkeit, vor allem aber wegen der nicht zu vernachlässigenden Aktivität von nicht imprägnierten Aluminiumoxid-Trägern zur Bildung von Nebenprodukten, nicht. Auch eine zu große Verdünnung der Aktivkomponenten auf dem Trägermaterial ist nicht günstig, da dadurch das Anspringen der Reaktion stark beeinträchtigt wird.

Aufgabe der Erfindung ist es, ein Verfahren zur Oxychlorierung von Ethen an einem Festbett-Kontakt zur Verfügung zu stellen, das auch bei hohen Reaktor-Belastungen ohne die Gegenwart von zusätzlichen Partikeln aus einem inerten Verdünnungsmaterial auskommt.

Gelöst wird die Aufgabe durch ein Verfahren zur Herstellung von 1,2-Dichlorethan durch Oxychlorierung von Ethen in Gegenwart eines kupferhaltigen Festbett-Kontakes aus einer Schüttung, im wesentlichen bestehend aus Katalysatorpartikeln, welche zumindest teilweise mit einer Aktivkomponente und gegebenenfalls einem Promotor imprägniertes Trägermaterial enthalten, wobei die Katalysatorschüttung im wesentlichen kein separates Inertmaterial zur Verdünnung enthält.

Ein separates Inertmaterial ist ein von den Katalysatorpartikeln verschiedenes, nicht imprägniertes oder nicht imprägnierbares, chemisch inertes Verdünnungsmaterial. In den Katalysatorpartikeln enthaltenes Inertmaterial ist im Sinne der vorliegenden Erfindung kein separates Inertmaterial. "Im wesentlichen bestehend aus Katalysatorpartikeln" heißt, daß die Schüttung zu mindestens 80 Gew.-%, vorzugsweise zu mindestens 90 Gew.-%, besonders bevorzugt zu mindestens 95 Gew.-% und speziell zu 100 Gew.-% aus den Katalysatorpartikeln besteht.

Der Erfindung liegt der Gedanke zugrunde, die einzelnen Katalysatorpartikel der Katalysatorschüttung derart auszugestalten, daß sich eine Schüttung mit einem Verhältnis von Katalysatormasse zu Reaktorvolumen ergibt, das demjenigen Verhältnis einer mit Partikeln aus einem inerten Material verdünnten Katalysatorschüttung entspricht, so daß , auf die Verdünnung mit zusätzlichen Partikeln aus inertem Material verzichtet werden kann. Dies wird dadurch erreicht, daß die einzelnen Katalysatorpartikel eine derartige filigrane Form aufweisen oder daß die einzelnen Katalysatorpartikel inertes Verdünnungsmaterial in derartigen Anteilen enthalten, daß die Schüttung besagtes Verhältnis von aus dem Stand der Technik bekannten verdünnten Schüttungen aufweist.

Die Katalysatorpartikel enthalten zumindest teilweise mit einem oder mehreren Aktivkomponenten und gegebenenfalls einem oder mehreren Promotoren imprägniertes Trägermaterial. Geeignete imprägnierbare Trägermaterialien sind dem Fachmann bekannt und beispielsweise in Ullmanns Enzyklopädie der technischen Chemie, 4. Auflage 1975, Band 9, S. 429 beschrieben und umfassen Aluminiumoxide, Silicagel und Diatomeenerde. Bevorzugtes Trägermaterial sind Aluminiumoxide, beispielsweise γ-Al₂O₃. Der Katalysatorträger ist vorzugsweise mit einem Kupfersalz, beispielsweise mit Kupferchlorid als Aktivkomponente und einem üblichen Promotor, in den dem Fachmann bekannten Verhältnissen, beispielsweise wie in Ullmanns Enzyclopädie der technischen Chemie, 4. Auflage 1975, Band 9, S. 429 und der dort zitierten Literatur beschrieben, imprägniert. Bevorzugte Promotoren sind Salze der Alkali-, Erdalkali- und Seltenerdenmetalle, besonders bevorzugt Salze von Kalium, Cäsium und Magnesium, wie Kaliumchlorid oder Magnesiumchlorid. Der Katalysatorträger kann, abhängig von dem Verfahren, das bei der Imprägnierung angewandt wird, teilweise oder vorzugsweise vollständig imprägniert sein. Teilweise imprägniert ist der Träger, wenn er lediglich oberflächlich imprägniert ist.

Der Einsatz von separaten Graphit-Partikeln zur Verdünnung der Katalysatorschüttung läßt sich mit einer der beiden folgenden Varianten vermeiden. Selbstverständlich können diese beiden Varianten auch kombiniert werden. So können einerseits Katalysatorformkörper hergestellt werden, die eine Mischung aus einem Trägermaterial (z.B. Aluminiumoxid) und einem Inertmaterial (z.B. Graphit) enthalten. Nach anschließender Tränkung der Formkörper werden so Katalysatoren erhalten, die verglichen mit Formkörpern, die ausschließlich das entsprechende Trägermaterial enthalten, bei einem vergleichbaren Aktivkomponentengehalt (bezogen auf den Anteil des Trägermaterials) und einer vergleichbaren Belastung der Katalysatorschüttung keine weitere Verdünnung mit einem separaten Inertmaterial erforderlich machen. Solche mit einem Inertmaterial gemischten Träger lassen sich einfacher als das reine Inertmaterial in sehr druckverlustarmen Formen herstellen. Darüber hinaus wird durch die innige Durchmischung von Träger- und Inertmaterial ein besonders homogenes Temperaturprofil erreicht. Andererseits können in einer zweiten Variante Katalysatorformkörper ausgewählt werden, die sich dadurch auszeichnen, daß ihr Schüttgewicht so gering ist, daß die Konzentration an imprägniertem Trägermaterial im Reaktor auch ohne Verwendung von separatem Inertmaterial der Konzentration an imprägniertem Trägermaterial bei Verwendung herkömmlicher mit Graphit verdünnter Katalysatorschüttungen entspricht.

Die Katalysatorschüttung besteht im wesentlichen aus Partikeln enthaltend das zumindest teilweise imprägnierte Trägermaterial, wobei gemäß einer Ausführungsform der Erfindung zumindest ein Teil der Katalysatorpartikel eine derartige Geometrie aufweist, daß deren Rüttelgewicht vor dem Tränken mit der Aktivkomponente und gegebenenfalls den Promotoren 550 g/l nicht übersteigt.

Das erfindungsgemäße Verfahren kann, wie in Ullmanns Enzyklopädie der technischen Chemie, Band 9, S. 429 ff. beschrieben, in Rohrreaktoren durchgeführt werden, welche die Katalysatorschüttung enthalten. Das Verfahren kann einstufig oder mehrstufig durchgeführt werden. Mehrstufig kann das Verfahren derart durchgeführt werden, daß der benötigte Sauerstoff in Form von reinem Sauerstoff oder Luft in Teilströmen auf die mehreren Reaktoren verteilt zugegeben wird. Das Verfahren kann wahlweise mit Kreisgas oder in einfacher Fahrweise betrieben werden. Die Umsetzung erfolgt im allgemeinen bei einer Temperatur von 200 bis 300°C und einem Druck von 1 bis 4 bar.

In einer Ausführungsform des erfindungsgemäßen Verfahrens bestehen die einzelnen Katalysatorpartikel aus dem zumindest teilweise imprägnierten Trägermaterial und weisen eine derartige Form auf, daß sich ein Hohlraumvolumen der resultierenden Schüttung von 45 bis 80, vorzugsweise von 55 bis 70, besonders bevorzugt von 60 bis 65 Vol.-% ergibt.

Die Katalysatorpartikel weisen vorzugsweise eine der folgenden Formen auf:
- Hohlzylinder;
- Zylinder mit axialen Bohrungen;
- Zahnrad mit einer oder mehreren axialen Bohrungen;
- koaxiale Hohlzylinder mit Stegen, welche den äußeren mit dem inneren Hohlzylinder verbinden (Wagenrad);
- Ringtabletten;
- kalottierte Ringtabletten;
- Trilobe;
- Tetralobe.

Eine bevorzugte Form der Katalysatorpartikel ist ein Zylinder mit axialen Bohrungen. Vorzugsweise weist der Zylinder ein Durchmesser-zu-Höhe-Verhältnis von 0,8 : 1 bis 1,2 : 1, insbesondere von 1 : 1 auf, wobei Durchmesser und Höhe der Zylinder jeweils vorzugsweise von 4 bis 8 mm variieren. Die Zylinder weisen vorzugsweise bis zu 5, insbesondere von 2 bis 4 axiale Bohrungen auf.

Die erfindungsgemäß eingesetzten Katalysatorpartikel können beispielsweise die Form eines Zylinders mit einer Bohrung (Hohlzylinder), einem Durchmesser : Höhe-Verhältnis von 0,8 : 1 bis 1,0 : 1 und einem Verhältnis von Zylinderdurchmesser : Durchmesser der Bohrung von 1,5 : 1 (=Durchmesser-Verhältnis) aufweisen. Solche Katalysatorpartikel weisen ein Hohlraumvolumen von 60,8 - 68,7 Vol.-% auf.

Die Katalysatorpartikel können beispielsweise zwei Bohrungen, einem Durchmesser : Höhe-Verhältnis von 0,8 : 1 bis 1:1 und ein Durchmesser-Verhältnis von 2,3 : 1 aufweisen. Solche Katalysatorpartikel weisen ein Hohlraumvolumen von 56,2 - 64,9 Vol.-% auf.

Die Katalysatorpartikel können beispielsweise 3 Bohrungen mit einem Durchmesser : Höhe-Verhältnis von 0,8 : 1 bis 1 : 1 und einem Durchmesser-Verhältnis von 2,5 : 1 aufweisen. Das Hohlraumvolumen beträgt dann 63,3 - 70,7 Vol.-%.

Die Katalysatorpartikel können beispielsweise 4 Bohrungen mit einem Durchmesser : Höhe-Verhältnis von 0,8 : 1 bis 1:1 und einem Durchmesser-Verhältnis von 3,0 : 1 aufweisen. Das Hohlraumvolumen beträgt dann 60,8 - 68,7 Vol.-%.
Ein weiteres Beispiel ist ein Zylinder mit den Abmessungen 7 x 7 mm und vier äquidistanten axialen Bohrungen mit einem Durchmesser von 1,75 mm.

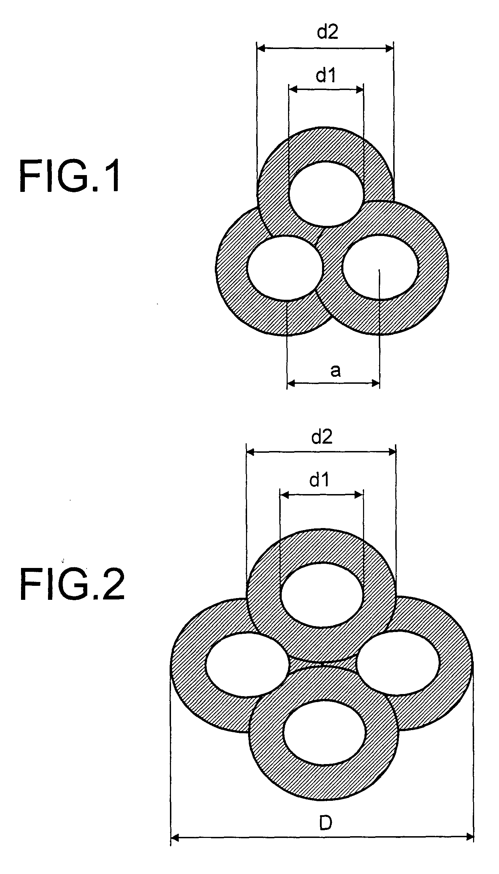

Die erfindungsgemäß eingesetzten Katalysatorpartikel können beispielsweise die in Figur 1 dargestellte Form eines Trilobes aufweisen. Dabei beträgt das Verhältnis d2/a vorzugsweise von 1,0 : 1 bis 1,4 : 1 und das Verhältnis d2/d1 vorzugsweise von 1,4 - 1,8 : 1. Ein Trilobe mit d2/a = 1,2 : 1 und d2/d1 = 1,6 : 1 weist ein Hohlraumvolumen von 62,7 Vol.-% auf.

Die erfindungsgemäß eingesetzten Katalysatorpartikel können beispielsweise die in Figur 2 dargestellte Form eines Tetralobes aufweisen. D kann beispielsweise von 4 bis 10 mm, d1 von 1,2 bis 3,0 mm und d2 = D/2 betragen, wobei bei einem Durchmesser D: Höhe-Verhältnis von 1:1 das Hohlraumvolumen 67,7 Vol.-% beträgt. Mit Durchmesser D = Höhe, d2 = D/2 und d1 = D/2 - D/K beträgt das Hohlraumvolumen für K = 2, 3, 4, 5 und 10 gleich 42,3 Vol.-%, 50,1 Vol.-%, 60,1 Vol.-%, 67,7 Vol.-% bzw. 87,4 Vol.-%.

Eine weitere bevorzugte Form der Katalysatorpartikel ist ein Zahnrad mit vorzugsweise zentraler Bohrung. Das Zahnrad ist vorzugsweise regelmäßig, das heißt es wird von einem Kreis umschrieben, wobei die Spitzen der Zähne die Kreislinie berühren. Das Verhältnis von Kreisdurchmesser des umschreibenden Kreises D zu Durchmesser des Innenkreises der Zähne Dᵢ beträgt vorzugsweise von 1,2 : 1 bis 1,8 : 1, wobei das Verhältnis von Kreisdurchmesser D zu Durchmesser der Bohrung dᵢ jeweils vorzugsweise von 1,8 : 1 bis 2,4 : 1 variiert. Das Zahnrad hat vorzugsweise von 5 bis 10, insbesondere 7 Zähne.

Ein Beispiel ist ein Zahnrad mit 7 Zähnen mit D/Dᵢ = 1,6 und D/dᵢ = 2,2 mit einem Hohlraumvolumen von 65,6 Vol.-%.

Eine weitere bevorzugte Form der Katalysatorpartikel ist die eines Wagenrads.

Ein Beispiel ist ein Wagenrad mit einem Durchmesser des Außenringes von 25 mm und einem Durchmesser des Innenringes von 9 mm mit 6 Stegen, wobei die Wandstärke der Stege jeweils 1,0 mm und diejenige der Ringe (Hohlzylinder) jeweils 1,5 mm beträgt. Ein solches Wagenrad weist ein Hohlraumvolumen von 62,3 Vol.-% auf.

Ein weiteres Beispiel ist ein Wagenrad mit einem Außenring von 8 mm Durchmesser, einem Innenring von 1,6 mm Durchmesser, 5 Stegen von 0,6 mm Wandstärke und einer Wandstärke der Ringe von 0,8 mm. Solche Wagenräder weisen als Schüttgut ein Hohlraumvolumen von 61,7 Vol.-% auf.

In einer weiteren Ausführungsform des erfindungsgemäßen Verfahrens enthält die Schüttung Katalysatorpartikel, die ein nicht imprägnierbares, chemisch inertes Verdünnungsmaterial enthalten. Vorzugsweise besteht die Katalysatorschüttung aus solchen Katalysatorpartikeln.

Die das inerte Verdünnungsmaterial enthaltenden Katalysatorpartikel enthalten im allgemeinen von 5 bis 80 , bevorzugt von 5 bis 60 , besonders bevorzugt von 10 bis 40 Gew.-% des inerten Verdünnungmaterials. Bevorzugte inerte Verdünnungsmaterialien sind feinteiliger Graphit, gemahlener Quarz oder Cordierit, beispielsweise in Form von Graphitpulver, Quarzsand oder Cordierit.

Gegenstand der vorliegenden Erfindung sind auch die das inerte Verdünnungsmaterial enthaltenden Katalysatorpartikel selbst.

Die das inerte Verdünnungsmaterial enthaltenden Katalysatorpartikel können alle üblichen Formen aufweisen. Prinzipiell sind druckverlustarme Formkörper bevorzugt. Beispiele sind Kugeln, Stränge, Tabletten und Ringe. Sie können ebenso die obenstehend definerten Formen eines Hohlzylinders, eines Zylinders mit mehreren axialen Bohrungen, eines Zahnrads, eines Wagenrads, eines Tri- oder Tetralobes aufweisen.

Die das inerte Verdünnungsmaterial enthaltenden Katalysatorpartikel können hergestellt werden in einem Verfahren umfassend die Schritte:
i) Mischen von imprägnierbarem Trägermaterial, inertem Verdünnungmaterial und gegebenenfalls üblichen Extrudier- bzw. Tablettierungshilfsmitteln;
ii) Tablettiern oder Extrudiern des erhaltenen Gemischs;
iii) Trocknen und Calcinieren der erhaltenen Tabletten oder Extrudate;
iv) Imprägnieren der Tabletten oder Extrudate mit der Aktivkomponente und gegebenenfalls den Promotoren.

Trägermaterial, inertes Verdünnungsmaterial und gegebenenfalls übliche Tablettierungshilfsmittel werden in dem Fachmann bekannter Weise gemischt und zu Katalysatorpartikeln tablettiert oder extrudiert. Das Calcinieren erfolgt bei Temperaturen von vorzugsweise 400 bis 800°C. Die so erhaltenen calcinierten Katalysatorpartikel werden anschließend nach üblichen Methoden, beispielsweise durch Tränken oder Aufsprühen, mit einer Kupferchlorid und den Promotor enthaltenden wäßrigen oder alkoholischen Lösung imprägniert. Es kann sich ein Trocknungsschritt anschließen. Die Imprägnierung kann so durchgeführt werden, daß die Lösung auf den erhitzten Katalysatorträger aufgesprüht wird, wobei das Lösungsmittel verdampft, bevor die Lösung in den Katalysatorpartikel eindringen kann. Dadurch kann eine nur oberflächliche, also teilweise Imprägnierung des Trägermaterials erreicht werden. Das Trägermaterial kann auch vollständig imprägniert werden.

Durch den Verzicht auf die Verdünnung der Katalysatorschüttung mit zusätzlichen Partikeln aus Inertmaterial ergibt sich ein sehr niedriger Druckverlust im Reaktor. Der dadurch bedingte erhöhte Stoff- und Wärmetransport und das resultierende homogene Temperaturprofil erlauben entweder höhere Belastungen und damit bessere Raum-Zeit-Ausbeuten oder, bei gleichbleibender Belastung, höhere Selektivitäten.

## Patentansprüche

1. Verfahren zur Herstellung von 1,2-Dichlorethan durch Oxychlorierung von Ethen in Gegenwart eines kupferhaltigen Festbett-Kontaktes aus einer Schüttung, im wesentlichen bestehend aus Katalysatorpartikeln, welche zumindest teilweise mit einer Aktivkomponente und gegebenenfalls einem Promotor imprägniertes Trägermaterial enthalten, wobei die Katalysatorschüttung im wesentlichen kein separates Inertmaterial zur Verdünnung enthält.

2. Verfahren nach Anspruch 1, dadurch gekennzeichnet, daß zumindest ein Teil der Katalysatorpartikel eine derartige Form aufweisen, daß deren Rüttelgewicht vor dem Tränken mit der Aktivkomponente und gegebenenfalls den Promotoren 550 g/l nicht übersteigt.

3. Verfahren nach Anspruch 1 oder 2, dadurch gekennzeichnet, daß die Schüttung Katalysatorpartikel enthält, deren Form ausgewählt ist aus:
- Hohlzylinder;
- Zylinder mit axialen Bohrungen;
- Zahnrad mit einer oder mehreren axialen Bohrungen;
- koaxiale Hohlzylinder mit Stegen, welche den äußeren mit dem inneren Hohlzylinder verbinden (Wagenrad);
- Ringtabletten;
- kalottierte Ringtabletten;
- Trilobe;
- Tetralobe.

4. Verfahren nach einem der Ansprüche 1 bis 3, dadurch gekennzeichnet, daß die Schüttung Katalysatorpartikel enthält, die zusätzlich ein nicht imprägnierbares, chemisch inertes Verdünnungsmaterial enthalten.

5. Verfahren nach Anspruch 4, dadurch gekennzeichnet, daß das inerte Verdünnungsmaterial Graphit, feinteiliger Quarz, Steatit oder Cordierit ist.

6. Verfahren nach einem der Ansprüche 1 bis 5, dadurch gekennzeichnet, daß die Aktivkomponente ein Kupfersalz ist.

7. Verfahren nach einem der Ansprüche 1 bis 6, dadurch gekennzeichnet, daß das imprägnierbare Trägermaterial ein Aluminiumoxid ist.

8. Verfahren nach einem der Ansprüche 1 bis 7, dadurch gekennzeichnet, daß der Promoter ein Kalium-, Cäsium- oder Magnesiumsalz ist.

9. Katalysatorpartikel, wie sie in einem der Ansprüche 4 bis 8 definiert sind.

10. Verfahren zur Herstellung von Katalysatorpartikeln, wie sie in einem der Ansprüche 4 bis 8 definiert sind, mit den Schritten:
v) Mischen von imprägnierbarem Trägermaterial, inertem Verdünnungsmaterial und gegebenenfalls üblichen Extrudier- bzw. Tablettierhilfsmitteln;
vi) Tablettiern oder Extrudiern des erhaltenen Gemischs;
vii) Trocknen und Calcinieren der erhaltenen Tabletten oder Extrudate;
viii) Imprägnieren der Tabletten oder Extrudate mit der Aktivkomponente und gegebenenfalls den Promotoren.
